# DEMANDE DE BREVET EUROPEEN

(11) **EP 3 018 523 A1**
(43) Date de publication de la demande: **11.05.2016**
(21) Numéro de dépôt: 15192964.3
(22) Date de dépôt: 04.11.2015
(51) Int. Cl.: G02B 27/01

(54) **SYSTEME DE VISUALISATION DE TETE COMPORTANT UN SYSTEME OCULOMETRIQUE ET DES MOYENS D'ADAPTATION DES IMAGES EMISES**

(30) Priorité: 07.11.2014 FR 1402529
(71) Demandeur: THALES, 92400 Courbevoie (FR)
(72) Inventeur: LUX, Johanna, 33187 LE HAILLAN Cedex (FR); ELLERO, Sébastien, 47170 ANDIRAN (FR); AUGEREAU, Philippe, 33187 LE HAILLAN Cedex (FR)
(74) Mandataire: Bréda, Jean-Marc

(57) **Abrégé**

Le domaine général de l'invention est celui des systèmes de visualisation de tête (1) destiné à être porté par un utilisateur. Ledit système de visualisation peut être monoculaire ou binoculaire. Il comporte un ou deux dispositifs de visualisation (10), un ou deux oculomètres (30) et des moyens de génération graphique (20). Chaque dispositif de visualisation comprend un afficheur (11), une optique de collimation (12) et un combineur optique (13) permettant de superposer une image issue de l'afficheur sur un paysage extérieur. Dans le système selon l'invention, le ou les oculomètres comportent des moyens de détermination de l'angle de convergence des deux yeux de l'utilisateur et de l'accommodation résultante. Le ou les optiques de collimation comportent des moyens permettant de projeter les images issues du ou des afficheurs à une distance fonction de ladite accommodation. Les moyens de génération graphique permettent d'afficher lesdites images à des positions fonction de ladite accommodation.

## Description

Le domaine de l'invention est celui des systèmes de visualisation portés par la tête d'un utilisateur. Ces systèmes permettent en particulier l'affichage d'informations en superposition sur le paysage extérieur. On les connaît notamment sous le terme anglais de « See-through HMD », « HMD » étant l'acronyme de « Head Mounted Display ». Ils peuvent être monoculaires ou binoculaires. Ils sont utilisés dans différentes applications. On citera leur utilisation dans les cockpits d'aéronefs civils et militaires où ils sont utilisés pour présenter au pilote des informations essentielles concernant le pilotage ou la navigation.

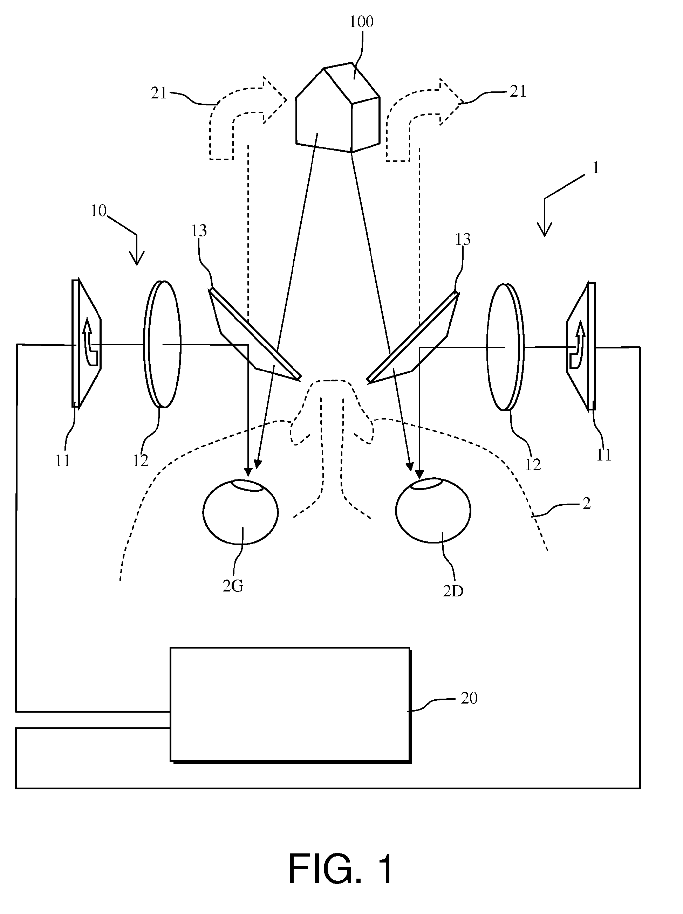

En général, ces HMD affichent des informations virtuelles à une distance donnée et fixe de l'utilisateur. Cette méthode présente plusieurs inconvénients :
- la différence d'accommodation entre l'image virtuelle et un objet réel du paysage extérieur sur lequel l'image virtuelle est superposée indique à l'utilisateur deux distances différentes pour deux éléments qui paraissent superposés ;
- L'utilisateur ne peut voir net ces deux éléments simultanément ;
- dans certaines configurations, cette situation crée un conflit de perception entre l'accommodation et la perception. L'accommodation indique que l'objet réel est proche de l'utilisateur et que l'image virtuelle est plus éloignée, alors que l'indice monoculaire de perception de l'espace qu'est l'occultation ou la superposition indique que l'image virtuelle est plus proche que l'objet réel puisqu'elle n'est pas cachée par celui-ci ;
- Lorsque le système est binoculaire, la différence de distance entre les images virtuelles et les objets réels entraîne un phénomène bien connu sous le nom de diplopie. Ce problème est illustré sur les figures 1, 2 et 3 et détaillé ci-dessous.

Lorsqu'une personne regarde un objet, chaque oeil accommode sur l'objet pour le voir net, par déformation du cristallin. Simultanément, les deux yeux convergent vers l'objet. Le cerveau fusionne les deux images provenant de l'oeil gauche et de l'oeil droit afin de voir un seul objet non dédoublé.

La figure 1 représente un HMD binoculaire 1. Il comporte deux dispositifs de visualisation 10 disposés devant les yeux 2G et 2D de l'utilisateur 2. Chaque dispositif comporte un afficheur 11 de petites dimensions, une optique de collimation 12 et un combineur optique 13 permettant de superposer une image issue de l'afficheur sur le paysage extérieur. Des moyens de génération graphique 20 affichent la même image sur les deux afficheurs. Dans le cas de la figure 1, l'image 21 est une flèche courbe blanche. Le paysage extérieur est symbolisé par une maison stylisée 100. Les flèches noires indiquent la propagation des rayons lumineux.

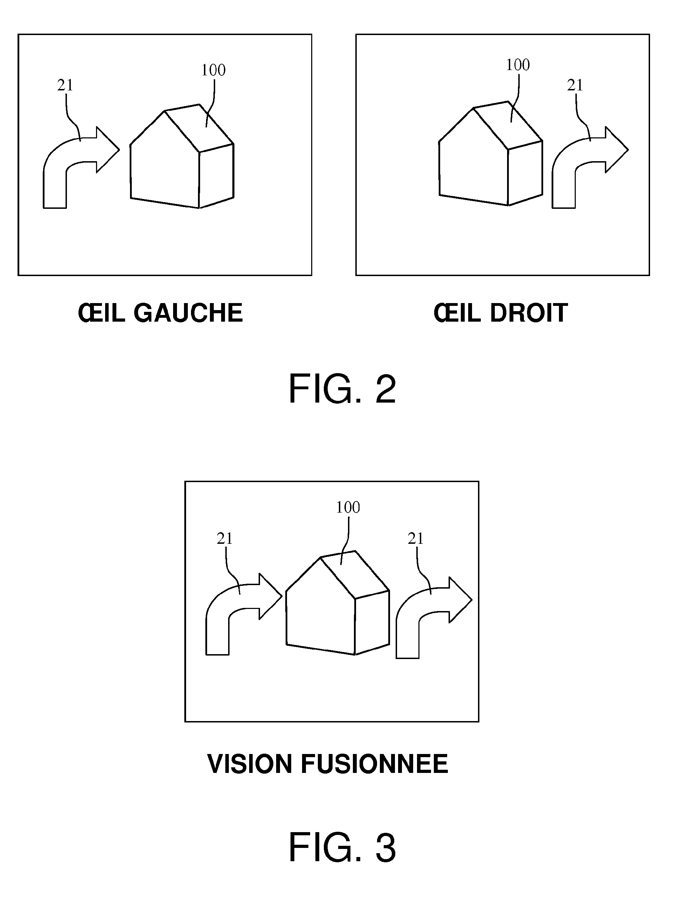

Lorsqu'une image virtuelle est présentée à l'utilisateur en mode binoculaire dans le HMD binoculaire 1, il peut y avoir une différence entre la distance de projection de l'image qui est classiquement à l'infini optique pour des applications aéronautiques et la distance réelle de l'élément du paysage extérieur sur lequel l'image est affichée en superposition. Ainsi, sur la figure 1, la maison 100 est à distance finie alors que la flèche est à l'infini optique. Comme on le voit sur la figure 2, dans ce cas, l'oeil gauche voit la flèche de l'afficheur à gauche de la maison et l'oeil droit voit la flèche de l'afficheur à droite de la maison. En vision fusionnée, si le cerveau privilégie le paysage extérieur, l'observateur verra, comme on le voit sur la figure 3, une seule maison et deux flèches. Si, à l'inverse, le cerveau privilégie les images issues des afficheurs, il verra une seule flèche et deux maisons. Dans tous les cas, l'objet réel et l'image virtuelle ne peuvent pas être vus non dédoublés et nets en même temps.

Bien entendu, les différences angulaires entre les images perçues sont plus faibles que ce qui est représenté schématiquement sur les figures 2 et 3. En effet, la distance interpupillaire humaine est d'environ 65 millimètres alors que les distances entre l'utilisateur et les objets extérieurs se comptent au minimum en mètres. En aéronautique, par exemple, les objets réels sont nécessairement à une distance certaine de l'aéronef comme on peut le comprendre. Cependant, un écart angulaire même faible entre l'image réelle et l'image virtuelle peut occasionner, à la longue, des troubles de la vision ou des fatigues visuelles importantes, d'autant plus que l'utilisateur n'en a pas nécessairement conscience.

Pour résoudre ces différents problèmes, différentes solutions techniques ont été proposées. Ainsi, la demande US 2013/0088413 intitulée « Method to Autofocus on Near-Eye Display » décrit un système de visualisation dans lequel la distance de l'image virtuelle est réglable, cette distance étant fonction de la distance des objets réels du paysage extérieur. Cette dernière distance est déterminée soit par un système d'autofocus comme on en trouve sur les caméras et les appareils-photos ou par un système de mesure de distance fonctionnant par émission de signaux optiques ou d'ultrasons. Cependant, ces différents moyens permettant de déterminer les distances des différents objets réels du paysage extérieur ne permettent pas de connaître quel objet est effectivement regardé par l'utilisateur.

La demande US 2013/0241805 intitulée « Using convergence angle to select among différent UI elements » décrit un système de visualisation comprenant un oculomètre, connu également sous le terme anglais de «Eye-tracker » permettant de déterminer l'angle de convergence entre les deux yeux de l'utilisateur, d'en déduire les objets regardés et d'afficher des images virtuelles en conséquence. Cependant, l'image virtuelle reste toujours affichée à la même distance.

Ainsi, ces deux solutions techniques ne permettent pas de résoudre totalement le problème posé par la superposition d'une image virtuelle sur un objet réel.

Le système de visualisation de tête selon l'invention combine et complète les deux systèmes précédents de façon à obtenir un système qui corrige au mieux et simplement à la fois les problèmes d'accommodation et de fusion binoculaire. Il n'y a alors plus de conflit de perception visuelle entre le paysage et les informations affichées par le système de visualisation. L'observateur voit net l'image ou les images du HMD et le paysage extérieur, sans dédoublement d'image. Plus précisément, l'invention a pour objet un système de visualisation de tête destiné à être porté par un utilisateur, ledit système de visualisation étant monoculaire et comportant au moins un dispositif de visualisation, un oculomètre et des moyens de génération graphique, le dispositif de visualisation comprenant un afficheur, une optique de collimation et un combineur optique permettant de superposer une image issue de l'afficheur sur un paysage extérieur, caractérisé en ce que :
l'oculomètre comporte des moyens de détermination de l'angle de convergence des deux yeux de l'utilisateur et de l'accommodation résultante ;
l'optique de collimation comporte des moyens permettant de projeter l'image issue de l'afficheur à une distance fonction de ladite accommodation ;
les moyens de génération graphique permettent d'afficher ladite image à une position fonction de ladite accommodation.

Avantageusement, ledit système est binoculaire et comporte au moins un second dispositif de visualisation, le second dispositif de visualisation comprenant un second afficheur, une seconde optique de collimation et un second combineur optique permettant de superposer une seconde image issue du second afficheur sur le paysage extérieur, la seconde optique de collimation comportant des moyens permettant de projeter l'image issue du second afficheur à une distance fonction de ladite accommodation et les moyens de génération graphique permettent d'afficher la seconde image à une position fonction de ladite accommodation.

Avantageusement, ledit système de visualisation comporte un second oculomètre, les deux oculomètres comportant des moyens de détermination de l'angle de convergence des deux yeux de l'utilisateur et de l'accommodation résultante.

Avantageusement, les informations issues du ou des oculomètres sont utilisées pour modifier les paramètres de l'image affichée.

Avantageusement, les informations issues du ou des oculomètres sont utilisées pour contrôler la vigilance de l'utilisateur.

Avantageusement, le système de visualisation de tête est un système aéronautique, l'utilisateur étant un pilote d'aéronef.

L'invention sera mieux comprise et d'autres avantages apparaîtront à la lecture de la description qui va suivre donnée à titre non limitatif et grâce aux figures annexées parmi lesquelles :
La figure 1 représente déjà commentée représente un système de visualisation de tête selon l'art antérieur ;
La figure 2 représente les images vues par l'oeil gauche et par l'oeil droit d'un utilisateur d'un tel système ;
la figure 3 représente la vision binoculaire de l'utilisateur d'un tel système ;
La figure 4 représente un système de visualisation de tête selon l'invention ;
La figure 5 représente les images vues par l'oeil gauche et par l'oeil droit d'un utilisateur d'un tel système selon l'invention ;
La figure 6 représente la vision binoculaire de l'utilisateur d'un tel système.

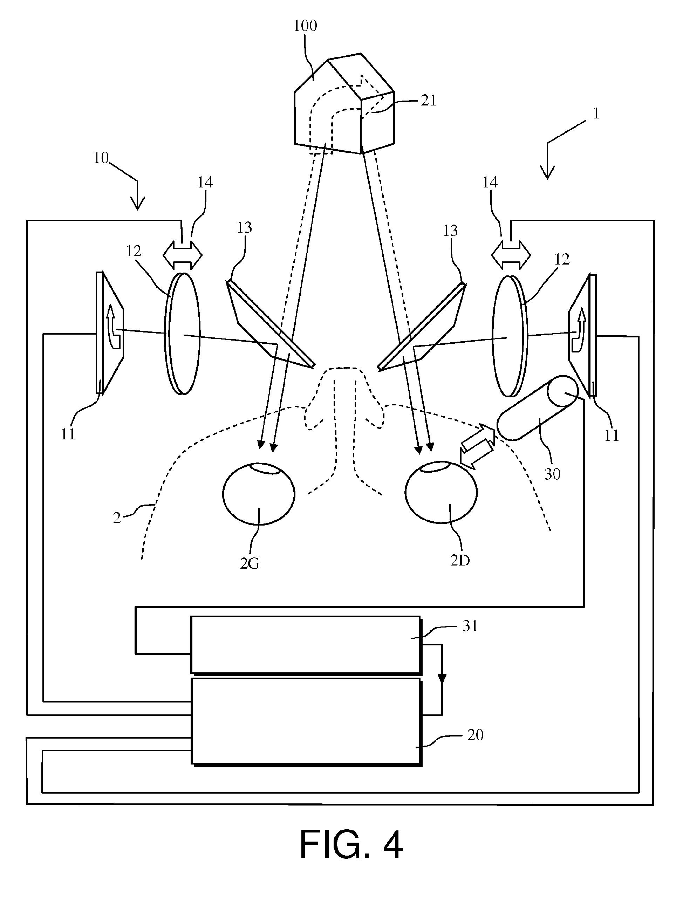

Le système de visualisation selon l'invention peut être monoculaire ou binoculaire. L'application principale du système selon l'invention est l'aide au pilotage d'aéronef. On peut utiliser un tel système pour toutes les applications nécessitant la superposition d'images synthétiques sur l'extérieur. La possibilité de réaliser des systèmes de visualisation de tête à des coûts réduits permet d'envisager une grande variété d'applications, tant dans le système des transports que des applications professionnelles nécessitant une vision à courte distance ou grand public. A titre d'exemple non limitatif, la figure 4 représente un système de visualisation binoculaire. Il est possible, sans difficultés techniques, de réaliser un système monoculaire à partir de ce premier système binoculaire.

Dans ce qui suit, on a repris les mêmes références que celles de la figure 1. Les flèches noires indiquent la propagation des rayons lumineux. Le système de visualisation comporte :
- deux dispositifs de visualisation 10 disposés devant les yeux 2G et 2D de l'utilisateur 2. Chaque dispositif comporte :
   o un afficheur 11 de petites dimensions qui peut être, par exemple, un afficheur à cristaux liquides ;
   o une optique de collimation 12. Sur la figure 4, les optiques de collimation sont représentées par de simples lentilles. Généralement, ces optiques sont des systèmes optiques complexes comportant plusieurs lentilles ou groupes de lentilles. Sur la figure 4, les flèches noires indiquent la propagation des rayons lumineux ;
   o un combineur optique 13 permettant de superposer une image issue de l'afficheur sur le paysage extérieur. Sur la figure 4, le combineur est symbolisé par une lame à faces planes et parallèles. Le combineur peut avoir d'autres formes, avoir de la puissance optique ou encore être intégré à une visière unique ;
   o des moyens de réglage 14 permettant de régler la distance de projection de l'image à une valeur déterminée. Ces moyens sont symbolisés par des flèches doubles sur la figure 4. Généralement, ces moyens consistent à déplacer d'une courte distance soit l'afficheur par rapport à l'optique de collimation, soit tout ou partie de l'optique de collimation par rapport à l'afficheur. Les moyens de déplacement peuvent être purement mécaniques ou électro-mécaniques. Il est également possible de changer un paramètre optique d'une lentille ou d'un miroir comme son indice optique ou son rayon de courbure ;
- Des moyens de génération graphique 20 affichent la même image sur les deux afficheurs. Ces moyens permettent d'afficher une symbologie représentative de paramètres de navigation ou de pilotage ou toute autre information concernant l'appareil. Dans le cas de la figure 4, l'image 21 est une flèche courbe blanche. Le paysage extérieur est symbolisé par une maison stylisée 100. Ces moyens pilotent également les moyens de réglage précédents ;
- Un oculomètre ou « eye-tracker » 30. La fonction de ce système est de repérer la direction du regard de l'utilisateur. Il existe différents principes de réalisation de ce type de système. Généralement, on utilise les réflexions sur la cornée et/ou sur la pupille de l'oeil pour calculer l'angle de convergence. Il est à noter que les optiques et les trajets optiques de l' « eye-tracker » et du HMD peuvent être en partie communs. Ainsi, l'oculomètre comprend une ou plusieurs sources d'éclairage, généralement situées dans le proche infrarouge pour ne pas perturber la vision, des moyens de photoréception de type micro-caméras et des moyens d'analyse 31 permettant de calculer les informations de convergence. Cette information est envoyée aux moyens de génération graphique 20. Il est à noter qu'il est possible de disposer un second oculomètre sur l'autre oeil, soit pour affiner la mesure, soit pour assurer sa redondance.

Comme il existe une synergie entre l'accommodation et la convergence des yeux, il est possible de déduire l'effort d'accommodation et donc la distance de l'objet observé uniquement à partir de la mesure de l'angle de convergence des yeux obtenue grâce à ou aux « eye trackers », Ainsi, la distance entre l'utilisateur et l'élément du paysage extérieur qui est regardé est connue.

Connaissant ces deux informations d'accommodation et de convergence, les moyens de génération graphique ajustent, en temps réel, à la fois :
- les deux distances de projection des images virtuelles afin qu'elles paraissent à la même distance que l'élément extérieur regardé ;
- les positions respectives des images affichées sur les deux afficheurs de façon que les images virtuelles projetées convergent toutes deux sur l'objet réel.

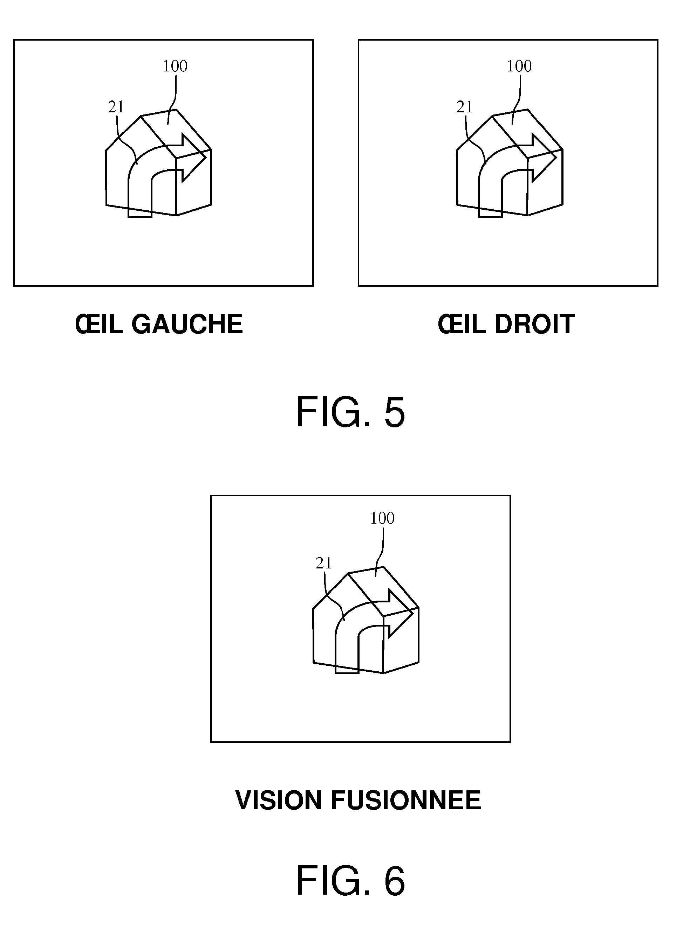

Comme on le voit sur les figures 5 et 6, dans ce cas, l'oeil gauche, l'oeil droit et la vision fusionnée voient la flèche 21 de l'afficheur parfaitement superposée à la maison représentant le paysage extérieur. Dans ce cas, les problèmes de flou et de dédoublement d'images ont disparu.

L'utilisation d' « eye-tracker » présente d'autres avantages. Ainsi, L'eye tracker peut servir de moyen d'interaction avec le HMD. Ainsi, on peut modifier les paramètres de l'image affichée en fonction de la connaissance de l'objet regardé. Un autre avantage est que l'eye tracker peut servir de moyen de contrôle de la vigilance de l'utilisateur. Cette fonction peut être particulièrement utile pour toutes les applications de pilotage.

Il est également possible de compléter le système de visualisation de tête selon l'invention par un système de détection de posture permettant de parfaitement connaître la posture de la tête de l'utilisateur par rapport à un référentiel connu.

Il existe différentes techniques permettant de repérer un objet dans l'espace. On peut utiliser la détection électromagnétique. Un émetteur est disposé dans le repère fixe et un récepteur dans le repère mobile. On peut également utiliser la détection optique qui peut être passive ou active. Dans ce dernier cas, le dispositif de visualisation porte des diodes électroluminescentes dont la position de l'émission est repérée par des caméras. Toutes ces techniques sont connues de l'homme du métier. Elles sont compatibles d'un fonctionnement en temps réel et s'adaptent facilement au système de visualisation selon l'invention.

## Revendications

1. Système de visualisation de tête (1) destiné à être porté par un utilisateur, ledit système de visualisation étant monoculaire et comportant au moins un dispositif de visualisation (10), un oculomètre (30, 31) et des moyens de génération graphique (20), le dispositif de visualisation comprenant un afficheur (11), une optique de collimation (12) et un combineur optique (13) permettant de superposer une image issue de l'afficheur sur un paysage extérieur, **caractérisé en ce que** :
l'oculomètre (30) comporte des moyens de détermination de l'angle de convergence des deux yeux de l'utilisateur et de l'accommodation résultante ;
l'optique de collimation (12) comporte des moyens (14) permettant de projeter l'image issue de l'afficheur à une distance fonction de ladite accommodation ;
les moyens de génération graphique (20) permettent d'afficher ladite image à une position fonction de ladite accommodation.

2. Système de visualisation de tête selon la revendication 1, **caractérisé en ce que** ledit système est binoculaire et comporte au moins un second dispositif de visualisation, le second dispositif de visualisation comprenant un second afficheur, une seconde optique de collimation et un second combineur optique permettant de superposer une seconde image issue du second afficheur sur le paysage extérieur, la seconde optique de collimation comportant des moyens permettant de projeter l'image issue du second afficheur à une distance fonction de ladite accommodation et les moyens de génération graphique permettant d'afficher la seconde image à une position fonction de ladite accommodation.

3. Système de visualisation de tête selon la revendication 2, **caractérisé en ce que** ledit système de visualisation comporte un second oculomètre, les deux oculomètres comportant des moyens de détermination de l'angle de convergence des deux yeux de l'utilisateur et de l'accommodation résultante.

4. Système de visualisation de tête selon l'une des revendications précédentes, **caractérisé en ce que** les informations issues du ou des oculomètres sont utilisées pour modifier les paramètres de l'image affichée.

5. Système de visualisation de tête selon l'une des revendications précédentes, **caractérisé en ce que** les oculomètres sont des moyens de contrôle de la vigilance de l'utilisateur.

6. Système de visualisation de tête selon l'une des revendications précédentes, **caractérisé en ce que** le système de visualisation de tête est un système aéronautique, l'utilisateur étant un pilote d'aéronef.
